# EUROPEAN PATENT SPECIFICATION

(11) **EP 2 520 318 B1**
(45) Date of publication and mention of the grant of the patent: **18.12.2013**
(21) Application number: 12166439.5
(22) Date of filing: 02.05.2012
(51) Int. Cl.: A61M 5/20

(54) **Adapter for syringes and associated dispensing devices and methods**
Adapter für Spritzen und dazugehörige Ausgabevorrichtungen und Verfahren
Adaptateur pour seringues et dispositifs et procédés de distribution associés

(30) Priority: 06.05.2011 US 201113102257
(43) Date of publication of application: 07.11.2012
(73) Proprietor: Nordson Corporation, Westlake, Ohio 44145-1119 (US)
(72) Inventor: Pappalardo, Matthew E., Ewing, NJ New Jersey 08618 (US); Springhorn, Robert W., Cream Ridge, NJ New Jersey 08514 (US)
(74) Representative: Eisenführ, Speiser & Partner

(56) References cited:
- EP-A1- 0 100 369
- WO-A1-2009/021020
- DE-A1-102006 005 784
- US-A1- 2009 018 512

## Description

### Technical Field

This invention generally relates to dispensing devices for dispensing a fluid from a syringe, and particularly to an adapter for coupling a syringe and an actuator.

### Background

In certain applications it is sometimes necessary to dispense liquids out of a syringe or a similar container. For example, hot melt adhesives may be dispensed out of a syringe-like cartridge and onto a desired target. Conventional dispensing systems generally include a syringe containing fluid and an actuator adapted to move a piston or plunger in the syringe to dispense the fluid. Syringes for these applications generally include a flange at a proximal end adapted to be coupled to the actuator.

However, conventional syringes are formed in a plurality of shapes and sizes for different fluids. Some conventional syringes include an annular flange, while other conventional syringes include a flange with arcuate portions separated by straight portions called flats. Many conventional syringes have flanges that conform to a set of industry-standard sizes and shapes. However, some companies produce syringes with specialized flanges having shapes and/or sizes not conforming to industry standards. Conventional actuators are designed to couple to one particular size and shape of flange on a syringe, such as the actuator described in WO 2009/021020 A1. From EP 0 100 369 A1 actuators are also known, which can be coupled to syringes with flanges of different sizes. Thus, every time a new syringe is to be used having a different shape or a different shape and a different size, a different actuator must be provided.

Additionally, some conventional syringes include finger grips and an elongate plunger adapted to be manually pressed to dispense the fluid from the syringe. In certain fluid application fields, manual actuation of dispensing from a syringe may not be optimal in all circumstances due to hand fatigue and strain. But conventional non-manual actuators generally cannot be coupled to syringes with finger grips. Again, a different specialized actuator must be provided for non-manual actuation of these conventional syringes with finger grips.

Similarly, in some fluid application fields it may be desirable to use manual actuation to dispense fluid from a syringe because manual actuators are quickly ready for use and easy to operate. A manual actuator does not require connection to a power source or an air supply, for example. However, it can be difficult to readily switch between a non-manual actuator and a manual actuator for a particular syringe because each actuator is adapted to couple to one particular size and shape of flange on a syringe.

For a professional seeking to dispense various kinds of fluid by manual and/or non-manual actuation, a plurality of actuators must be purchased and maintained to accommodate the various shapes and sizes of flanges and finger grips on syringes. Consequently, multiple couplers or actuators still must be available in order to dispense various kinds of fluid from syringes having different shapes and sizes by manual and/or non-manual actuation.

There is a need, therefore, for an adapter that addresses these and other problems associated with conventional dispensing systems.

### Summary

The invention is defined in claims 1 and 15. In one embodiment of the invention, an adapter is adapted to couple an actuator to a syringe including an elongate body and a flange. The adapter includes a housing with a proximal end wall, a distal end wall, and a side wall extending between the proximal and distal end walls and defining an outer peripheral surface. The distal end wall is adapted to face toward the syringe. The adapter also includes a first slot extending inwardly from the outer peripheral surface and adapted to couple with a syringe flange. The first slot defines a first shape and a first size. The adapter further includes a second slot extending inwardly from the outer peripheral surface and adapted to couple with a syringe flange. The second slot defines a second shape different than the first shape and a second size different than the first size.

The first or second slot may be U-shaped to closely couple with flanges having a circular shape or flats. The first or second slot may also be adapted to closely couple with flanges including finger grip portions. In some embodiments, the first slot may be positioned in the housing at least partially coextensive with the second slot. Alternatively, the first slot and the second slot may be separated by a divider wall including an internal aperture sized to receive the elongate body of the syringe. The internal aperture includes a retention portion and a seat portion, the retention portion adapted to retain the elongate body of the syringe in the seat portion when the syringe is coupled to the adapter. Similarly, one or more of the slots may also include a retention portion and a seat portion, the retention portion adapted to retain the flange of a syringe in the seat portion when the syringe is coupled to the adapter. The adapter may include additional slots extending inwardly from the outer peripheral surface to accommodate even more types of syringe flanges. Thus, the adapter is advantageously adapted to couple many different types of syringes to a single actuator.

In another embodiment of the invention, a dispensing device for dispensing fluid contained in a syringe includes an actuator and an adapter. The syringe includes an elongate body and a flange. The adapter includes a housing with a proximal end wall, a distal end wall, and a side wall extending between the proximal and distal end walls and defining an outer peripheral surface. The proximal end wall includes an aperture. The distal end wall is adapted to face toward the syringe. The adapter also includes a first slot extending inwardly from the outer peripheral surface and adapted to couple with a syringe flange. The first slot defines a first shape and a first size. The adapter further includes a second slot extending inwardly from the outer peripheral surface and adapted to couple with a syringe flange. The second slot defines at least one of a second shape different than the first shape and a second size different than the first size. The actuator is coupled to the adapter and includes a plunger adapted to be advanced in a distal direction through the aperture in the proximal end wall to push fluid contained in the syringe.

In yet another embodiment of the invention, a method of dispensing fluid utilizes a dispensing device including an actuator having a plunger and an adapter. The adapter includes a housing with a first slot defining a first shape and a first size and a second slot defining at least one of a second shape different than the first shape and a second size different than the first size. The method includes inserting a flange of a first syringe into the first slot of the adapter to operatively couple the first syringe to the actuator. The method also includes moving the plunger of the actuator to dispense fluid from the first syringe, and removing the flange of the first syringe from the first slot to decouple the first syringe from the adapter. The method further includes inserting a flange of a second syringe into the second slot of the adapter to operatively couple the second syringe to the actuator, and moving the plunger of the actuator to dispense fluid from the second syringe.

In another embodiment of the invention, an adapter for coupling an actuator to a syringe includes a housing and a first slot. The syringe includes an elongate body and a flange. The housing includes a proximal end wall, a distal end wall, and a side wall extending between the proximal and distal end walls. The side wall defines an outer peripheral surface. The proximal end wall is adapted to face toward the actuator and the distal end wall is adapted to face toward the syringe. The first slot extends inwardly from the outer peripheral surface and defines a first shape and a first size. The first slot is adapted to closely couple with a syringe flange including finger grip portions.

Various additional features and advantages of the invention will become more apparent upon review of the following detailed description of the illustrative embodiments taken in conjunction with the accompanying drawings.

### Brief Description of the Drawings

FIG. 1 is a perspective view of a dispensing device including one embodiment of an adapter according to the current invention.

FIG. 2 is a perspective view of the dispensing device of FIG. 1, with the actuator and the syringe shown partially in phantom.

FIG. 3 is a perspective view of the adapter of FIG. 1.

FIG. 4A is a cross-sectional side view of the adapter of FIG. 3 along line 4A-4A.

FIG. 4B is a cross-sectional side view of the dispensing device of FIG. 1 along line 4B-4B, with a first syringe positioned in the adapter.

FIG. 4C is a cross-sectional side view of the dispensing device of FIG. 1 along line 4B-4B, with a second syringe positioned in the adapter.

FIG. 4D is a cross-sectional side view of the dispensing device of FIG. 1 along line 4B-4B, with a third syringe positioned in the adapter.

FIG. 4E is a cross-sectional side view of the dispensing device of FIG. 1 along line 4B-4B, with an alternative plunger of the actuator.

FIG. 4F is a cross-sectional front view of the adapter of FIG. 4A along line 4F-4F.

FIG. 5 is a perspective view of a dispensing device including another embodiment of an adapter according to the current invention.

FIG. 6 is a perspective view of the adapter of FIG. 5.

FIG. 7A is a cross-sectional side view of the adapter of FIG. 6 along line 7A-7A.

FIG. 7B is a cross-sectional side view of the adapter of FIG. 5 along line 7B-7B, with a first syringe positioned in the adapter.

FIG. 7C is a cross-sectional side view of the adapter of FIG. 5 along line 7B-7B, with a second syringe positioned in the adapter.

FIG. 7D is a cross-sectional side view of the adapter of FIG. 5 along line 7B-7B, with a third syringe positioned in the adapter.

FIG. 8 is a perspective view of a dispensing device including another embodiment of an adapter according to the current invention, with a first syringe positioned in the adapter.

FIG. 9 is a perspective view of the dispensing device of FIG. 8, with a second syringe positioned in the adapter.

FIG. 10 is a perspective view of the adapter of FIG. 8.

FIG. 11 is a cross-sectional side view of the adapter of FIG. 10 along line 11-11.

FIG. 12A is a cross-sectional side view of the adapter of FIG. 8 along line 12A-12A, with the first syringe positioned in the adapter.

FIG. 12B is a cross-sectional side view of the adapter of FIG. 9 along line 12B-12B, with the second syringe positioned in the adapter.

### Detailed Description

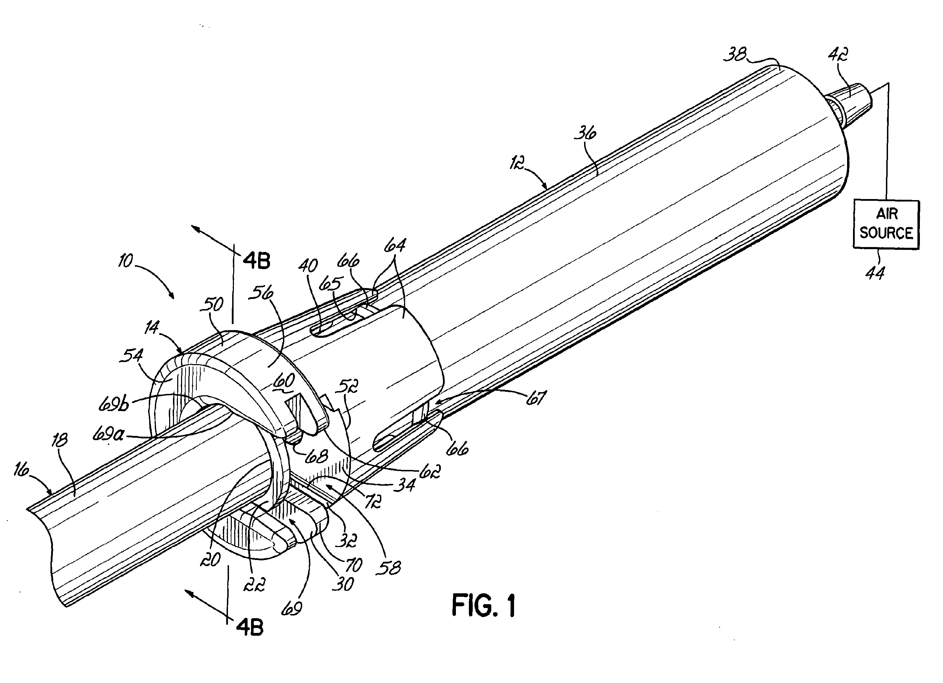

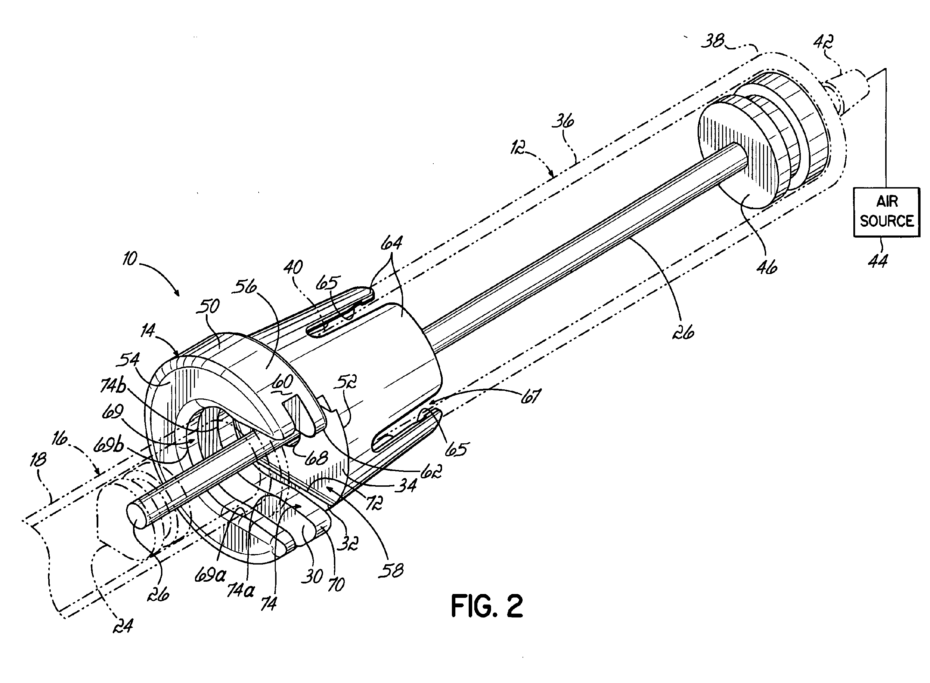

FIGS. 1-4D illustrate one embodiment of a dispensing device 10 according to one embodiment of the invention. The dispensing device 10 includes an actuator 12 and an adapter 14 adapted to couple the actuator 12 to a syringe 16 containing a fluid to be dispensed, such as hot melt adhesive or medicament. The syringe 16 includes an elongate body 18 with a proximal end 20 and a flange 22 located at the proximal end 20. The flange 22 may define any of a plurality of shapes and sizes depending on the manufacturer of the syringe 16. The syringe 16 also includes a movable piston 24 within the elongate body 18 and adapted to force the fluid out of the syringe 16, as best shown in FIG. 2. To this end, the actuator 12 includes a plunger 26 adapted to apply force on the piston 24 to move the piston 24 in the elongate body 18 and thereby dispense fluid from the syringe 16. The adapter 14 couples with the flange 22 of the syringe 16 to enable coupling of the actuator 12 and the syringe 16. Advantageously, the adapter 14 includes at least two slots 30, 32, 34 defining differing shapes and/or differing sizes so that the slots 30, 32, 34 may be coupled with syringe flanges having various shapes and sizes, thereby enabling ready coupling of different syringes to the actuator 12. Thus, the same actuator 12 may be used to dispense fluid from various types of syringes 16.

The actuator 12 illustrated in the embodiment shown in FIGS. 1 and 2 is a non-manual actuator 12, and more specifically, a pneumatic actuator 12. In this regard, the actuator 12 includes a housing 36 having a proximal end 38 and a distal end 40. Although the housing 36 is shown defining a cylindrical shape, other shapes of the housing 36 are possible within the scope of this invention. The proximal end 38 includes an inlet port 42 adapted to be connected to a pressurized air source 44. The actuator 12 of this embodiment also includes an actuation piston 46 connected to the plunger 26 and adapted to move along the housing 36. Consequently, when pressurized air is provided from the pressurized air source 44 through the inlet port 42, the pressurized air applies force to advance the actuation piston 46 and the plunger 26. As briefly described above, this advancement of the plunger 26 causes dispensing of fluid from the syringe 16 when the syringe 16 is coupled to the actuator 12 by the adapter 14. Although the actuator 12 shown in FIGS. 1 and 2 is a pneumatic actuator 12, other types of non-manual actuators and manual actuators may be used with the dispensing device 10. For example, the actuator 12 may be electrically powered such that the plunger 26 is driven by an electric motor such as a stepper motor in other embodiments.

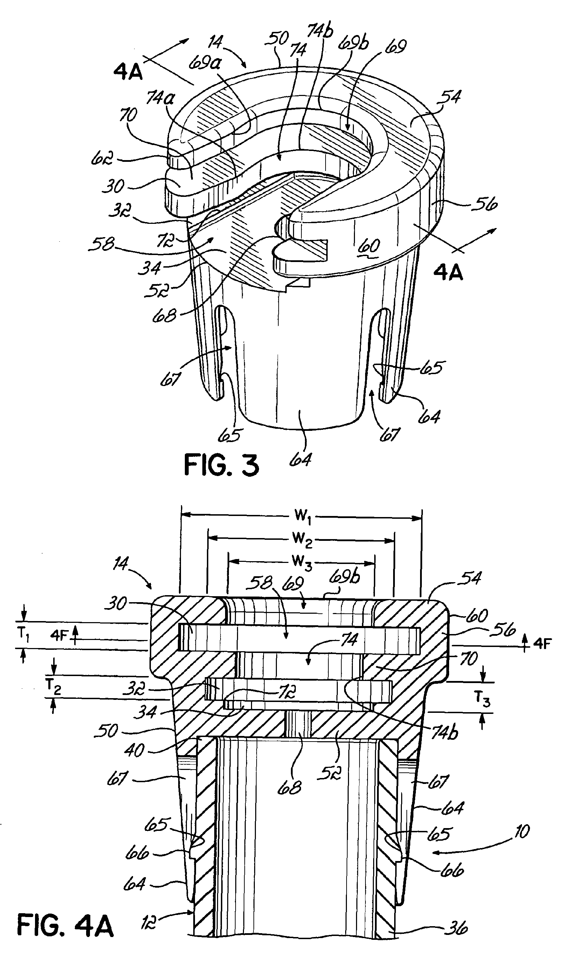

Further details of the adapter 14 of this embodiment are shown in FIGS. 3 and 4A. The adapter 14 includes a housing 50 including a proximal end wall 52 facing toward the actuator 12, a distal end wall 54 facing toward the syringe 16, and a side wall 56 extending between the proximal end wall 52 and the distal end wall 54. The housing 50 may be molded from a plastic material, machined from metal materials such as steel or aluminum, or formed from other known materials and manufacturing methods. The side wall 56 is adapted to at least partially enclose an interior 58 of the housing 50 defined between the proximal end wall 52 and the distal end wall 54. To this end, the side wall 56 defines an outer peripheral surface 60 of the housing 50. The adapter 14 further includes the first slot 30 and the second slot 32. The adapter 14 may also include the third slot 34. Each of the slots 30, 32, 34 extends inwardly into the interior 58 of the housing 50 from the outer peripheral surface 60 along one side 62 of the adapter 14. Each of the slots 30, 32, 34 is adapted to closely couple with a syringe flange having differing shapes and sizes.

The adapter 14 also includes a plurality of locking arms 64 extending proximally from the proximal end wall 52. Each of the locking arms 64 includes a locking notch 65 adapted to snap over a corresponding locking detent 66 formed near the distal end 40 of the actuator 12. To this end, each of the locking arms 64 is slightly resilient to permit snapping engagement of the locking arms 64 with the locking detent 66 on the actuator 12, as shown in FIG. 4A. The locking arms 64 are shaped to match the shape of the actuator 12, which is cylindrical in the illustrated embodiment. The locking arms 64 are separated by generally linear grooves 67 extending almost to the proximal end wall 52, but the grooves 67 may be omitted or shaped differently in other embodiments of the adapter 14. The locking arms 64 are shown as tapering in thickness away from the proximal end wall 52, but the locking arms 64 may alternatively define a constant thickness along their length. It will be understood that different known engagement mechanisms may be provided on the actuator 12 and the adapter 14, such as a rotatable snap-lock connection or a Luer-lock connection.

The proximal end wall 52 of the adapter 14 further includes an aperture 68 adapted to receive the plunger 26 of the actuator 12 when the actuator 12 is coupled to the adapter 14. The aperture 68 communicates with the distal end 40 of the actuator 12 and the interior 58 of the adapter 14, including each of the slots 30, 32, 34. The aperture 68 may be sized to closely receive the plunger 26 for movement through the adapter 14. Similarly, the distal end wall 54 of the adapter 14 further includes an opening 69 adapted to receive the elongate body 18 of the syringe 16 when the syringe 16 is coupled to the adapter 14. The opening 69 is generally U-shaped to permit passage of the elongate body 18 of the syringe 16 into the interior 58 from the side 62 of the adapter 14. The opening 69 communicates with the aperture 68 in the proximal end wall 52 and the interior 58 of the adapter 14, including each of the slots 30, 32, 34. Thus, the plunger 26 of the actuator 12 may freely pass through the aperture 68 in the proximal end wall 52, the interior 58, and the opening 69 in the distal end wall 54 to enter the elongate body 18 of the syringe 16.

The opening 69 in the distal end wall 54 of the adapter 14 further includes a retaining portion 69a and a seat portion 69b. The retaining portion 69a defines a smaller width or diameter than the seat portion 69b such that the retaining portion 69a snaps around the elongate body 18 of the syringe 16 when the elongate body 18 is positioned in the seat portion 69b of the opening 69. In the illustrated embodiment, the retaining portion 69a is defined by a pair of opposing detents positioned adjacent to the seat portion 69b. However, the retaining portion 69a may alternatively include any structure tending to retain the elongate body 18 of the syringe 16 in the seat portion 69b, such as a narrowed width or diameter neck portion extending outwardly from the seat portion 69b in one example. Thus, the retaining portion 69a deters inadvertent disconnection of the syringe 16 and the adapter 14.

As shown in FIGS. 3 and 4A, each of the slots 30, 32, 34 is generally U-shaped in this embodiment of the adapter 14. The first slot 30 extends between the distal end wall 54 of the adapter 14 and a divider wall 70 formed in the interior 58. The first slot 30 defines a first width W₁ and a first thickness T₁. In this regard, the first slot 30 defines a first shape and a first size adapted to closely couple with an annular or circular flange. The second slot 32 extends between the divider wall 70 and the proximal end wall 52 of the adapter 14. The second slot 32 defines a second width W₂ shorter than the first width W₁ and a second thickness T₂. Thus, the second slot 32 defines a second size different than the first size.

The third slot 34 also extends between the divider wall 70 and the proximal end wall 52 of the adapter 14 so as to be partially integral or coextensive with the second slot 32. However, the third slot 34 defines a third width W₃ shorter than the second width W₂ and a third thickness T₃ larger than the second thickness T₂. To this end, the third slot 34 and the second slot 32 collectively form a shoulder 72 between the slots 32, 34 in the proximal end wall 52. Furthermore, the third slot 34 defines a third shape different from the first shape and a third size different from the first and second sizes. As a result, the adapter 14 may be advantageously used to readily couple syringes 16 having at least three different types of flanges 22 to the actuator 12, thereby removing the need for separate actuators for each type of syringe.

The divider wall 70 further includes an internal aperture 74 communicating with each of the first, second, and third slots 30, 32, 34. The internal aperture 74 is sized to receive the elongate body 18 of syringes 16 adapted to engage the second or third slots 32, 34. To this end, the internal aperture 74 may be smaller in size (as shown in FIG. 4A) or equal in size to the opening 69 in the distal end wall 54 of the adapter 14. Similar to the opening 69, the internal aperture 74 includes a retaining portion 74a and a seat portion 74b, where the retaining portion 74a is adapted to retain the elongate body 18 of the syringe 16 in the seat portion 74b of the internal aperture 74. The retaining portion 74a may include detents adjacent the seat portion 74b as shown in the figures or may include alternative structure that narrows outwardly from the seat portion 74b. Although the first slot 30 and the second slot 32 are separated by the divider wall 70, the first and second slots 30, 32 may be at least partially coextensive in other embodiments of the adapter 14. Likewise, though the second slot 32 and the third slot 34 are shown as partially coextensive, the second and third slots 32, 34 may be divided by another divider wall in other embodiments of the adapter 14.

The adapter 14 illustrated in FIGS. 1-4A includes three slots 30, 32, 34. However, other embodiments of the adapter 14 may includes two slots for syringe flanges or more than three slots for syringe flanges depending on the needs of the user of the actuator 12. For example, the adapter 14 could be reconfigured with five slots for use by a doctor who works with five specific types of medicament syringes. Regardless of the number of slots 30, 32, 34 provided in the adapter 14, the slots 30, 32, 34 may be arranged in order of increasing size between the proximal end wall 52 and the distal end wall 54 such that the internal apertures 74 in any dividing walls 70 and the opening 69 in the distal end wall 54 may also be arranged in order of increasing size. Alternatively, the slots 30, 32, 34 may be reconfigured in any sequential order within the adapter 14 as required.

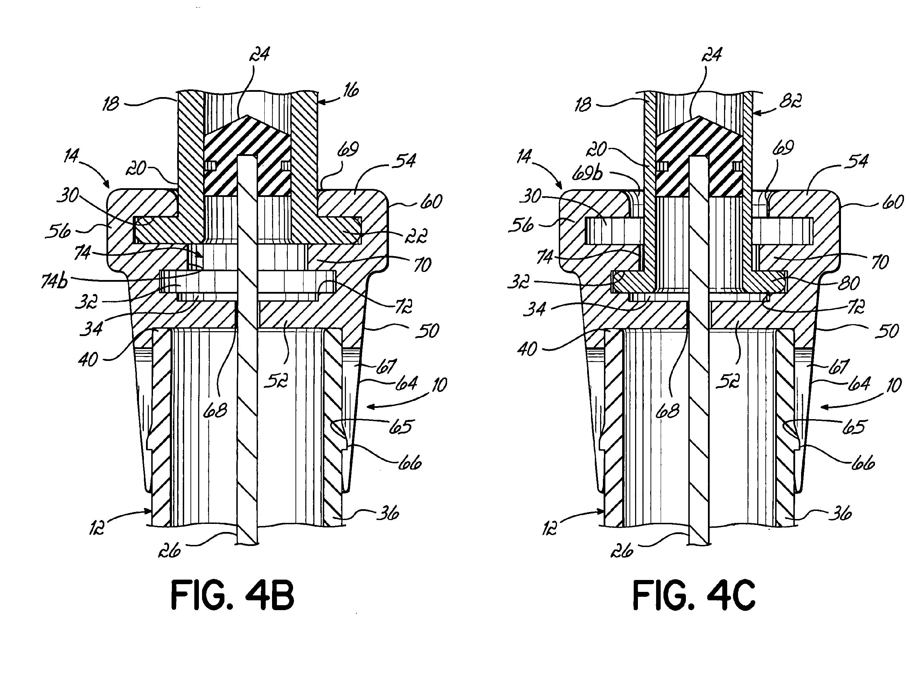

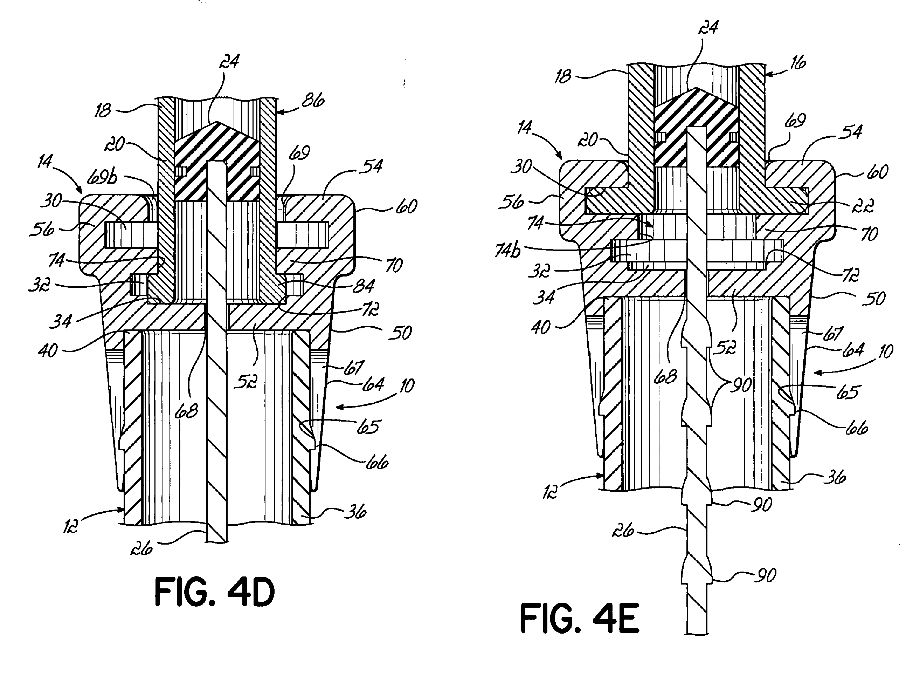

FIG. 4A illustrates the adapter 14 of the current embodiment without any syringe in position, while FIGS. 4B-4D show the adapter 14 engaged with various syringes. More particularly, the flange 22 of the syringe 16 shown in FIG. 1 may be inserted into and closely coupled with the first slot 30 as shown in FIG. 4B. Alternatively, a second annular flange 80 of a second syringe 82 may be inserted into and closely coupled with the second slot 32 as shown in FIG. 4C. Additionally, a third flange 84 (including flats) of a third syringe 86 may be inserted into and closely coupled with the third slot 34 as shown in FIG. 4D. Once the chosen syringe 16, 82, 86 is coupled to the actuator 12 by the adapter 14, the plunger 26 may be extended into engagement with the movable piston 24 within the chosen syringe 16, 82, 86. The actuator 12 may then be used to advance the plunger 26 and the movable piston 24 to dispense fluid from the syringe 16, 82, 86. Thus, the actuator 12 may be advantageously coupled to and used with syringes 16, 82, 86 of various shapes and sizes.

The adapter 14 and actuator 12 may collectively be configured for multiple uses with different syringes 16 or configured for a single use only. To this end, the plunger 26 may threadably couple to the movable piston 24 or may be inserted into an interference fit with a corresponding slot in the movable piston 24. Also as shown in FIG. 4E, the plunger 26 may include a plurality of barbs 90 along the length of the plunger 26. When the plunger 26 is advanced into the syringe 16, the barbs 90 engage with the proximal end wall 52 of the adapter 14 to prevent retraction of the plunger 26 from the adapter 14. Alternatively, the plunger 26 may be adapted to loosely engage the movable piston 24 such that the plunger 26 is fully retractable from the syringe 16 and the adapter 14 for re-use with another syringe 16.

The actuator 12, the adapter 14, and the syringe 16 are each illustrated as separate elements in the embodiment of FIGS. 1-4F. Alternatively, the actuator 12 and the adapter 14 may be a unitary structure in other embodiments so that the actuator 12 is always configured to couple with various particular types of syringes 16. In another alternative, a kit could be provided with one or more syringes 16 and the adapter 14. To this end, the syringe 16 may be combined with the adapter 14 into a unitary structure adapted to be coupled to one or more types of actuator 12. Consequently, the invention is not limited to the illustrated embodiment in which each of the actuator 12, the adapter 14, and the syringe 16 are separate elements.

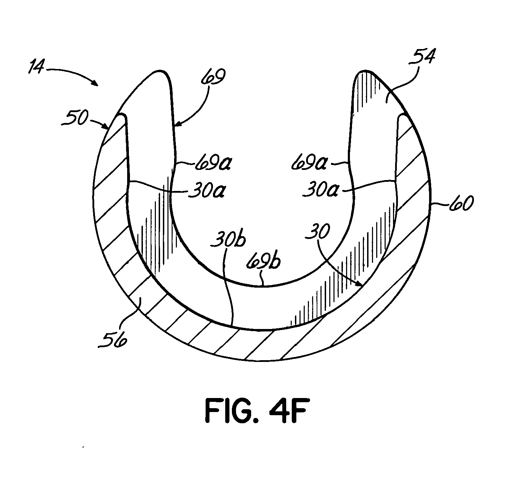

As shown in FIG. 4F, the first slot 30 of the adapter 14 further includes a retaining portion 30a and a seat portion 30b, similar to the opening 69 in the distal end wall 54. The retaining portion 30a defines a smaller width or diameter than the seat portion 30b such that the retaining portion 30a snaps around the flange 22 of the syringe 16 when the flange 22 is positioned in the seat portion 30b of the first slot 30. In the illustrated embodiment, the retaining portion 30a is defined by a pair of opposing detents positioned adjacent to the seat portion 30b. However, the retaining portion 30a may alternatively include any structure tending to retain the flange 22 of the syringe 16 in the seat portion 30b, such as a narrowed width or diameter neck portion extending outwardly from the seat portion 30b in one example. Thus, the retaining portion 30a of the first slot 30 and the retaining portion 69a of the opening 69 collectively deter inadvertent disconnection of the syringe 16 and the adapter 14. Although not shown in the drawings, it will be understood that any number of the slots 30, 32, 34 in this embodiment of the adapter 14 and other embodiments of the adapter may include corresponding retaining and seat portions to assist with retention of a syringe 16.

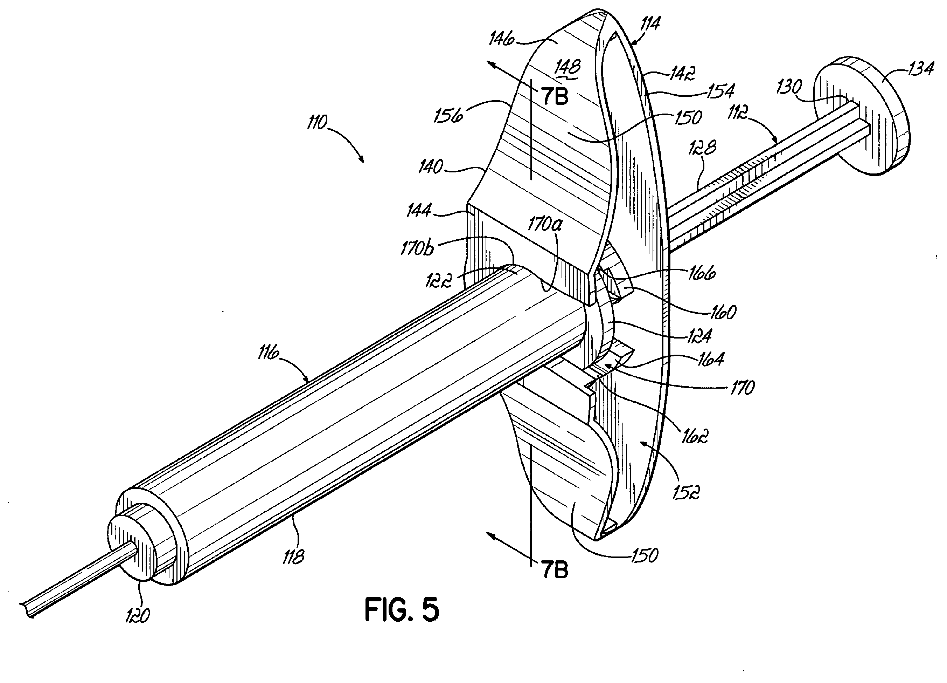

Another embodiment of a dispensing device 110 is shown in FIGS. 5-7D. The dispensing device 110 is similar to the previously-described embodiment of a dispensing device 10, with the differences highlighted below. The dispensing device 110 includes an actuator 112 and an adapter 114 adapted to assist with coupling the actuator 112 to a syringe 116 containing a fluid. Similar to the previous syringes discussed, the syringe 116 includes an elongate body 118 having a distal end 120 and a proximal end 122 with a flange 124 at the proximal end 122. The syringe 116 also includes a movable piston 126 adapted to apply force to the fluid within the elongate body 118 to dispense the fluid from the distal end 120. The actuator 112 of this embodiment is a manual actuator 112 including a plunger 128 having a proximal end 130 and a distal end 132 adapted to apply force to move the piston 126 within the syringe 116. The plunger 128 defines a cross-shaped cross section between the proximal end 130 and the distal end 132 in FIG. 5, but it will be appreciated that circular or other cross sections of the plunger 128 may be used within the scope of this invention. The actuator 112 also includes a thumb press 134 at the proximal end 130 of the plunger 128. Similar to the previous embodiment, the adapter 114 enables the operable coupling of differently sized and shaped syringes to the actuator 112.

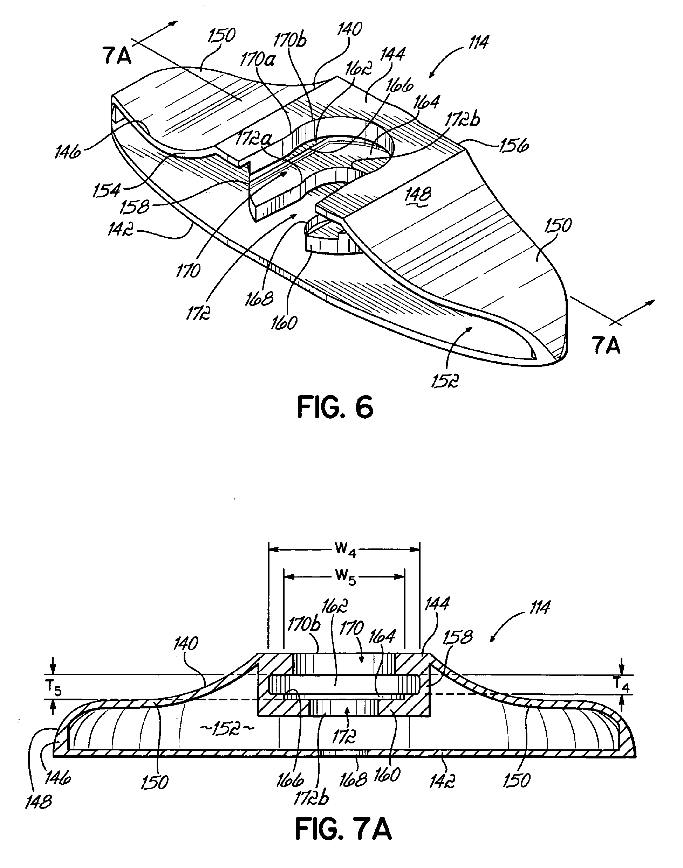

As shown in FIGS. 6 and 7A, the adapter 114 of this embodiment includes a housing 140 including a proximal end wall 142, a distal end wall 144, and a side wall 146 extending between the proximal end wall 142 and the distal end wall 144. The housing 140 may be molded from a plastic material or machined from a metal material, as described above. The side wall 146 projects outwardly from the distal end wall 144 to define an outer peripheral surface 148 including finger grip portions 150 or finger gripping surfaces along either side of the distal end wall 144. The proximal end wall 142, the distal end wall 144, and the side wall 146 collectively enclose an interior 152 of the adapter 114, the interior 152 extending from a first lateral side 154 of the adapter 114 to a second lateral side 156 of the adapter 114. The adapter 114 further includes an interior side wall 158 and an interior divider wall 160 collectively forming a first slot 162 and a second slot 164, each slot 162, 164 adapted to closely couple with syringe flanges having different shapes and sizes.

Each of the first and second slots 162, 164 is generally U-shaped in this embodiment of the adapter 114. The first slot 162 extends between the distal end wall 144 and the interior divider wall 160. The first slot 162 defines a first width W₄ and a first thickness T₄. In this regard, the first slot 162 defines a first shape and a first size and is adapted to closely couple with annular or circular syringe flanges. The second slot 164 also extends between the distal end wall 144 and the interior divider wall 160 so as to be partially integral or coextensive with the first slot 162. However, the second slot 164 defines a second width W₅ shorter than the first width W₄ and a second thickness T₅ larger than the first thickness T₄. To this end, the first slot 162 and the second slot 164 collectively form a shoulder 166 between the slots 162, 164 in the interior divider wall 160. Furthermore, the second slot 164 is adapted to closely couple with a flange having flats. The second slot 164 defines a second size different than the first size and a second shape different than the first shape. The first and second slots 162, 164 open at the first lateral side 154 of the adapter 114 so that the corresponding syringe flanges may be slid into the interior 152 of the adapter 114 and into engagement with one of the slots 162, 164.

The proximal end wall 142 of the adapter 114 further includes an aperture 168 adapted to receive the plunger 128 of the actuator 112 when the actuator 112 is coupled to the syringe 116. The aperture 168 communicates with the interior 152 of the adapter 114, including each of the slots 162, 164. The distal end wall 144 of the adapter 114 further includes an opening 170 adapted to receive the elongate body 118 of the syringe 116 when the syringe 116 is coupled to the adapter 114. The opening 170 is generally U-shaped to permit passage of the elongate body 118 of the syringe 116 into the interior 152 from the first lateral side 154 of the adapter 114. The opening 170 includes a retaining portion 170a and a seat portion 170b as previously described, where the retaining portion 170a is adapted to retain the elongate body 118 of the syringe 116 in the seat portion 170b of the internal aperture 170. The retaining portion 170a may include detents adjacent the seat portion 170b as shown in the figures or may include alternative structure that narrows outwardly from the seat portion 170b. The opening 170 communicates with the aperture 168 in the proximal end wall 142 and the interior 152 of the adapter 114, including each of the slots 162, 164.

Similarly, the interior divider wall 160 also includes an internal aperture 172 communicating with the aperture 168 in the proximal end wall 142 and the opening 170 in the distal end wall 144. The internal aperture 172 is generally U-shaped and sized to receive the elongate body 118 of the syringe 116. Similar to the opening 170, the internal aperture 172 includes a retaining portion 172a and a seat portion 172b, where the retaining portion 172a is adapted to retain the elongate body 118 of the syringe 116 in the seat portion 172b of the internal aperture 172. The retaining portion 172a may include detents adjacent the seat portion 172b as shown in the figures or may include alternative structure that narrows outwardly from the seat portion 172b. Consequently, the plunger 128 of the actuator 112 may freely pass through the aperture 168 in the proximal end wall 142, the interior 152, the internal aperture 172 in the interior divider wall 160, and the opening 170 in the distal end wall 144 to enter the elongate body 118 of the syringe 116. Although the aperture 168 is illustrated to be sized larger than the plunger 128 so that the plunger 128 may freely rotate with respect to the adapter 114, the aperture may be sized to slidably engage the plunger 128 in other embodiments.

In operation, the adapter 114 is coupled to the manual actuator 112, and the flange 124 of the syringe 116 is closely coupled with one of the slots 162, 164. A user may then grasp the adapter 114 at the finger grip portions 150 and the thumb press 134 of the actuator 112 to push the plunger 128 forwards toward the syringe 116. As the plunger 128 advances, the movable piston 126 within the syringe 116 is forced to move forward and push fluid out of the syringe 116. The thumb press 134 and plunger 128 may then be retracted so that a different syringe may be coupled with the adapter 114.

Although the illustrated adapter 114 includes two slots 162, 164, the adapter 114 may be reconfigured to include more than two slots in other embodiments. Furthermore, the slots 162, 164 may be separated by a divider wall rather than being partially coextensive in some embodiments. The finger grip portions 150 may also be removable from the remainder of the housing 140 of the adapter 114 in other embodiments. In such embodiments, when the adapter 114 is coupled to a non-manual actuator instead of a manual actuator 112, the finger grip portions 150 are removed because a user will not need the additional gripping surface to advance the plunger 128.

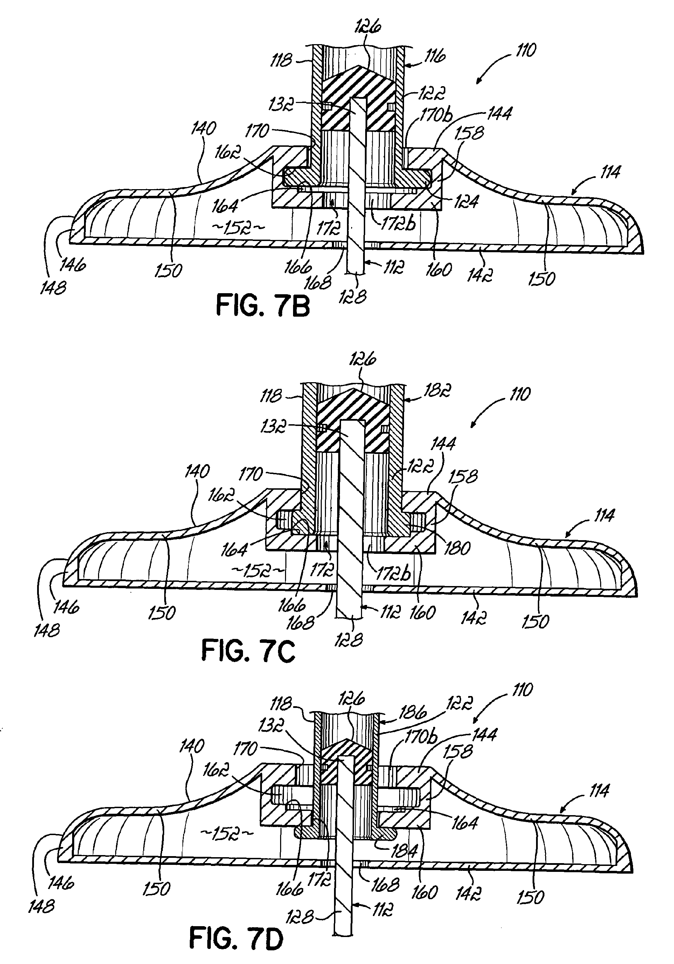

FIG. 7A illustrates the adapter 114 of the current embodiment without any syringe in position, while FIGS. 7B-7D show the adapter 114 engaged with various syringes. More particularly, the flange 124 of the syringe 116 shown in FIG. 5 may be inserted into and closely coupled with the first slot 162 as shown in FIG. 7B. Alternatively, a second flange 180 (including flats) of a second syringe 182 may be inserted into and closely coupled with the second slot 164 as shown in FIG. 7C. Additionally, a third flange 184 of a third syringe 186 may be inserted into the interior 152 of the adapter 114 behind the interior divider wall 160 as shown in FIG. 7D. Although the third flange 184 is not closely coupled in this position, the third flange 184 will stay in abutting relationship with the interior divider wall 160 as long as a force is being applied by the manual actuator 112. Once the chosen syringe 116, 182, 186 is coupled to the actuator 112 by the adapter 114, the plunger 128 may be extended into engagement with the movable piston 126 within the chosen syringe 116, 182, 186. The actuator 112 may then be used to advance the plunger 128 and the movable piston 126 to dispense fluid from the syringe 116, 182, 186. Thus, the actuator 112 may be advantageously coupled to and used with syringes 116, 182, 186 of various shapes and sizes.

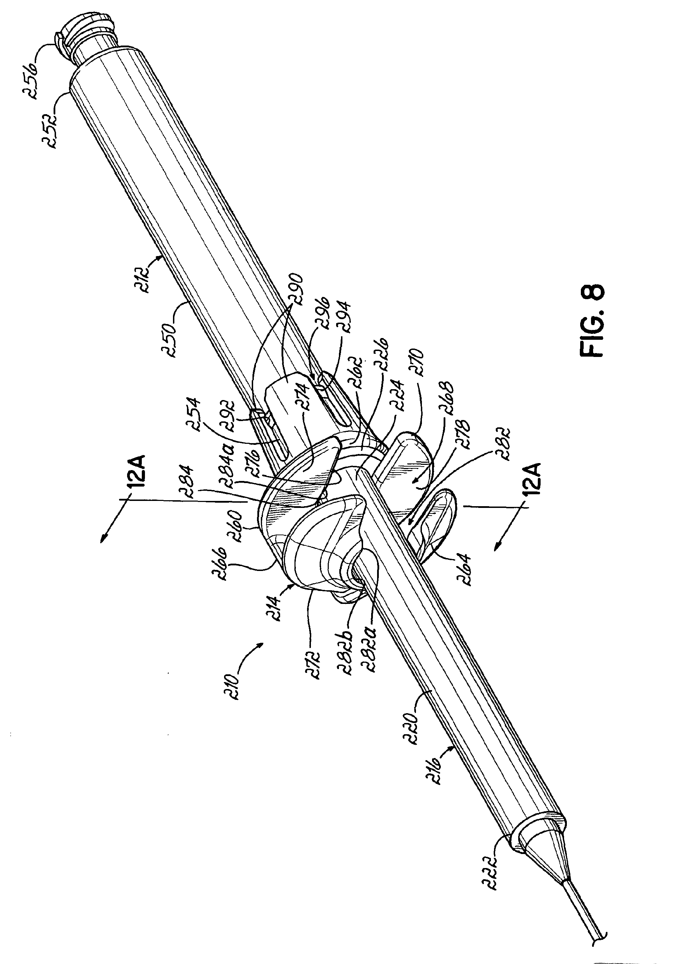

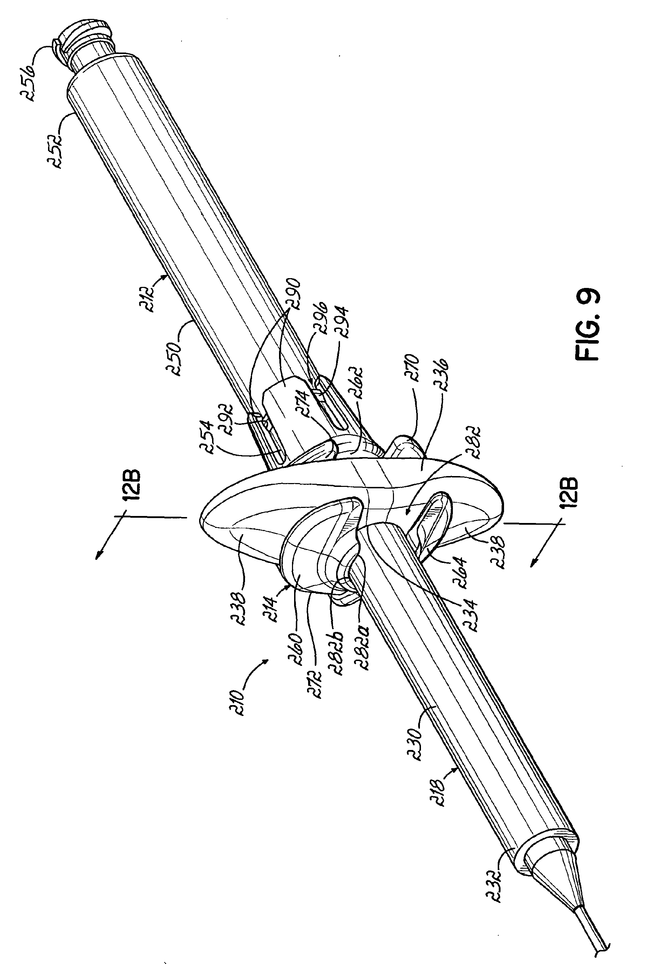

Another embodiment of a dispensing device 210 is shown in FIGS. 8-12B. The dispensing device 210 is similar to the previously-described embodiments of a dispensing device 10, 110, with the differences highlighted below. The dispensing device 210 includes an actuator 212 and an adapter 214 adapted to couple the actuator 212 to a first syringe 216 (FIG. 8) containing a fluid or a second syringe 218 (FIG. 9) containing a fluid. Similar to the previous syringes discussed, the first syringe 216 includes an elongate body 220 having a distal end 222 and a proximal end 224 with a flange 226 at the proximal end 224. The first syringe 216 also includes a movable piston 228 adapted to apply force to the fluid within the elongate body 220 to dispense the fluid from the distal end 222. Similarly, the second syringe 218 includes an elongate body 230 having a distal end 232 and a proximal end 234 with a finger flange 236 at the proximal end 234. The finger flange 236 of the second syringe 218 includes opposing finger grip portions 238 adapted to be grasped when the second syringe 218 is coupled to a manual actuator. The second syringe 218 also includes a movable piston 240 adapted to apply force to the fluid within the elongate body 230 to dispense the fluid from the distal end 232. Thus, the adapter 214 of this embodiment is adapted to couple syringes having regular flanges and finger flanges to the actuator 212.

The actuator 212 of this embodiment is a non-manual actuator, and more specifically, a pneumatic or mechanical actuator 212. In this regard, the actuator 212 includes a housing 250 having a proximal end 252 and a distal end 254. Although the housing 250 is shown defining a cylindrical shape, other shapes of the housing 250 are possible within the scope of this invention. The proximal end 252 includes an inlet port 256 adapted to be connected to a drive source such as a drive shaft or a pressurized air source. The actuator 212 also includes a plunger 258 adapted to be driven to move along the housing 250. Consequently, the plunger 258 is operable to apply force to move the movable piston 228, 240 of the syringe 216, 218 that is coupled to the actuator 212 by the adapter 214. It will be understood that other types of non-manual actuators and manual actuators may be used with the dispensing device 210 in other embodiments of the invention.

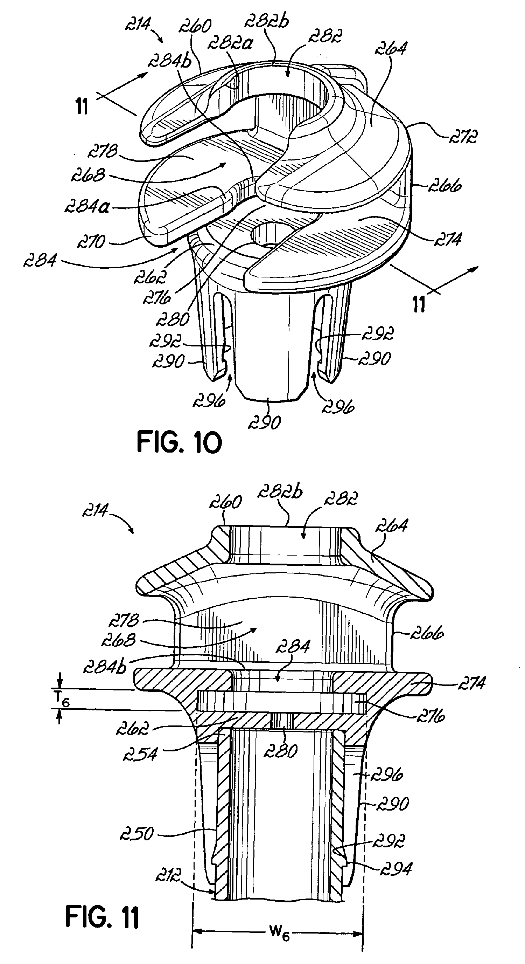

As shown in FIGS. 10 and 11, the adapter 214 of this embodiment includes a housing 260 including a proximal end wall 262, a distal end wall 264, and a side wall 266 extending between the proximal end wall 262 and the distal end wall 264. The housing 260 may be molded from a plastic material or machined from a metal material, as described above. The proximal end wall 262, the distal end wall 264, and the side wall 266 collectively enclose an interior 268 of the adapter 214, the interior 268 extending from a first lateral side 270 of the adapter 214 to a second lateral side 272 of the adapter 214. The adapter 214 further includes an interior divider wall 274 disposed between the proximal end wall 262 and the distal end wall 264. The adapter 214 includes a first slot 276 formed between the interior divider wall 274 and the proximal end wall 262 and a second slot 278 formed between the interior divider wall 274 and the distal end wall 264. The first slot 276 and the second slot 278 are adapted to closely couple with syringe flanges having different shapes and sizes.

The first slot 276 in the adapter 214 is generally U-shaped in this embodiment, opening at the first lateral side 270. The first slot 276 defines a first width W₆ and a first thickness T₆. In this regard, the first slot 276 is adapted to couple with an annular or circular flange. The first slot 276 defines a first shape and a first size. The second slot 278 extends through the entire side wall 266 except at the second lateral side 272 so that a finger flange 236 with finger grip portions 238 may be inserted into the second slot 278 with the finger grip portions 238 extending beyond the side wall 266 of the adapter 214. The second slot 278 is adapted to closely couple with a flange having finger grip portions. The second slot 278 defines a second shape different than the first shape and a second size different than the first size. Thus, the adapter 214 of this embodiment is adapted to closely couple with syringes having circular flanges or flanges with finger grip portions.

The proximal end wall 262 of the adapter 214 further includes an aperture 280 adapted to receive the plunger 258 of the actuator 212 when the actuator 212 is coupled to the adapter 214. The aperture 280 communicates with the interior 268 of the adapter 214, including each of the slots 276, 278. The distal end wall 264 of the adapter 214 further includes an opening 282 adapted to receive the elongate body 220, 230 of one of the syringes 216, 218. The opening 282 is generally U-shaped to permit passage of the elongate body 220, 230 into the interior 268 from the first lateral side 270 of the adapter 214. The opening 282 includes a retaining portion 282a and a seat portion 282b as previously described, where the retaining portion 282a is adapted to retain the elongate body 220, 230 of one of the syringes 216, 218 in the seat portion 282b of the internal aperture 282. The retaining portion 282a may include detents adjacent the seat portion 282b as shown in the figures or may include alternative structure that narrows outwardly from the seat portion 282b. The opening 282 communicates with the aperture 280 in the proximal end wall 262 and the interior 268 of the adapter 214, including each of the slots 276, 278.

Similarly, the interior divider wall 274 also includes an internal aperture 284 communicating with the aperture 280 in the proximal end wall 262 and the opening 282 in the distal end wall 264. The internal aperture 284 is generally U-shaped and sized to receive the elongate body 220, 230 of one of the syringes 216, 218. Similar to the opening 282, the internal aperture 284 includes a retaining portion 284a and a seat portion 284b, where the retaining portion 284a is adapted to retain the elongate body 220, 230 of one of the syringes 216, 218 in the seat portion 284b of the internal aperture 284. The retaining portion 284a may include detents adjacent the seat portion 284b as shown in the figures or may include alternative structure that narrows outwardly from the seat portion 284b. Consequently, the plunger 258 of the actuator 212 may freely pass through the aperture 280 in the proximal end wall 262, the interior 268, the internal aperture 284 in the interior divider wall 274, and the opening 282 in the distal end wall 264 to enter the elongate body 220, 230 of the corresponding syringe 216, 218.

The adapter 214 also includes a plurality of locking arms 290 extending proximally from the proximal end wall 262. Each of the locking arms 290 includes a locking notch 292 adapted to snap over a corresponding locking detent 294 formed near the distal end 254 of the actuator 212. To this end, each of the locking arms 290 is slightly resilient to permit snapping engagement of the locking arms 290 with the locking detent 294 on the actuator 212, as shown in FIG. 11. The locking arms 290 are shaped to match the shape of the actuator 212, which is cylindrical in the illustrated embodiment. The locking arms 290 are separated by generally linear grooves 296 extending almost to the proximal end wall 262, but the grooves 296 may be omitted or shaped differently in other embodiments of the adapter 214. The locking arms 290 are shown as tapering in thickness away from the proximal end wall 262, but the locking arms 290 may alternatively define a constant thickness along their length. It will be understood that different known engagement mechanisms may be provided on the actuator 212 and the adapter 214, such as a rotatable snap-lock connection or a Luer-lock connection.

Although the illustrated adapter 214 includes two slots 276, 278, the adapter 214 may be reconfigured to include more than two slots in other embodiments. These additional slots may be partially coextensive with one of the two slots 276, 278 or may be separated from these slots 276, 278 by a divider wall. Furthermore, the additional slots may be adapted to couple with syringe flanges having circular flanges, flanges with flats, flanges with finger grip portions, or flanges having other shapes and sizes as understood in the syringe art.

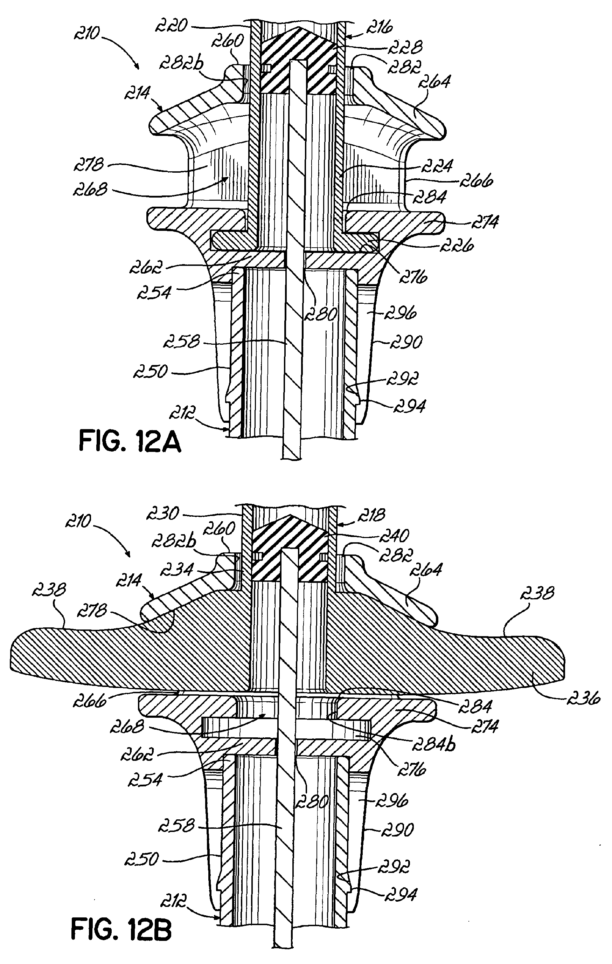

FIG. 11 illustrates the adapter 214 of the current embodiment without any syringe in position, while FIGS. 12A and 12B show the adapter 214 engaged with various syringes 216, 218. More particularly, the flange 226 of the first syringe 216 shown in FIG. 8 may be inserted into and closely coupled with the first slot 276 as shown in FIG. 12A. Alternatively, the second flange 236 (including finger grip portions 238) of the second syringe 218 may be inserted into and closely coupled with the second slot 278 as shown in FIG. 12B. Once the chosen syringe 216, 218 is coupled to the actuator 212 by the adapter 214, the plunger 258 may be extended into engagement with the movable piston 228, 240 within the chosen syringe 216, 218. The actuator 212 may then be used to advance the plunger 258 and the movable piston 228, 240 to dispense fluid from the syringe 216, 218. Thus, the actuator 212 may be advantageously coupled to and used with syringes 216, 218 of various shapes and sizes.

Although the illustrated adapter 214 includes two slots 276, 278, the adapter may also be reconfigured in other embodiments to include only the slot 278 adapted to receive finger flanges 236 including finger grip portions 238. The adapter of these other embodiments advantageously enables the coupling of a non-manual actuator to the syringe 220 having finger flanges 236, which normally is only actuated by manual actuators. Similar to the illustrated adapter 214, the adapter of these other embodiments allows the syringe 220 having finger flanges 236 to be readily coupled with non-manual actuators and manual actuators.

Further described are the following embodiments of examples:
Embodiment 1. An adapter for coupling an actuator to a syringe including an elongate body and a flange, the adapter comprising:
   a housing including a proximal end wall, a distal end wall, and a side wall extending between said proximal and distal end walls and defining an outer peripheral surface, said distal end wall adapted to face toward the syringe;
   a first slot extending inwardly from said outer peripheral surface and adapted to couple with a syringe flange, said first slot defining a first shape and a first size; and
   a second slot extending inwardly from said outer peripheral surface and adapted to couple with a syringe flange, said second slot defining at least one of a second shape different than said first shape and a second size different than said first size.
Embodiment 2. The adapter with the features of embodiment 1, wherein at least one of said first shape and said second shape is U-shaped so as to closely couple with circular syringe flanges.
Embodiment 3. The adapter with the features of embodiment 1, wherein at least one of said first shape and said second shape is U-shaped so as to closely couple with syringe flanges with flats.
Embodiment 4. The adapter with the features of embodiment 1, wherein at least one of said first shape and said second shape is adapted to closely couple with syringe flanges with finger grip portions.
Embodiment 5. The adapter with the features of embodiment 1, wherein said first slot is positioned in said housing so as to be at least partially coextensive with said second slot.
Embodiment 6. The adapter with the features of embodiment 1,
further comprising:
   a divider wall positioned between said first slot and said second slot, said divider wall including an internal aperture communicating with each of said first and second slots, said internal aperture adapted to receive the elongate body of the syringe.
Embodiment 7. The adapter with the features of embodiment 6, wherein said internal aperture includes a retention portion and a seat portion, said retention portion adapted to retain the elongate body of the syringe in said seat portion when the syringe is coupled to the adapter.
Embodiment 8. The adapter with the features of embodiment 1, further comprising:
   a third slot extending inwardly from said outer peripheral surface and adapted to couple with a syringe flange, said third slot defining a third shape different than said first shape and said second shape, a third size different than said first size and said second size, or both said third shape and said third size.
Embodiment 9. The adapter with the features of embodiment 8, wherein said first, second, and third slots are positioned in order of increasing size between said proximal end wall and said distal end wall of said housing.
Embodiment 10. The adapter with the features of embodiment 8, further comprising:
   a divider wall positioned between said first slot and said second slot, said divider wall including an internal aperture communicating with each of said first and second slots, said internal aperture adapted to receive the elongate body of the syringe,
   wherein said second slot is positioned in said housing so as to be partially coextensive with said third slot.
Embodiment 11. The adapter with the features of embodiment 1, wherein said proximal end wall of said housing includes an aperture adapted to receive a plunger of an actuator, and said distal end wall of said housing includes an opening in communication with said aperture and adapted to receive the elongate body of the syringe.
Embodiment 12. The adapter with the features of embodiment 11, wherein said opening includes a retention portion and a seat portion, said retention portion adapted to retain the elongate body of the syringe in said seat portion when the syringe is coupled to the adapter.
Embodiment 13. The adapter with the features of embodiment 11, wherein the outer peripheral surface of the housing further includes finger grip portions adapted to be grasped when the plunger of the actuator is manually actuated.
Embodiment 14. The adapter with the features of embodiment 11, wherein the outer peripheral surface of the housing further includes finger grip portions, the finger grip portions being removable from the housing when the plunger of the actuator is to be non-manually actuated.
Embodiment 15. The adapter with the features of embodiment 1, wherein at least one of said first and second slots includes a retention portion and a seat portion, said retention portion adapted to retain the flange of the syringe in said seat portion when the syringe is coupled to the adapter.
Embodiment 16. The adapter with the features of embodiment 1, wherein said proximal end wall is coupled to an actuator.
Embodiment 17. The adapter with the features of embodiment 16, wherein a flange of a syringe is coupled to one of said first slot or said second slot.
Embodiment 18. A dispensing device for dispensing fluid contained in a syringe having an elongate body and a flange, the dispensing device comprising:
   an adapter adapted to be coupled to the syringe and further comprising:
      a housing including a proximal end wall, a distal end wall, and a side wall extending between said proximal and distal end walls and defining an outer peripheral surface, said proximal end wall including an aperture, said distal end wall adapted to face toward the syringe;
      a first slot extending inwardly from said outer peripheral surface and adapted to couple with a syringe flange, said first slot defining a first shape and a first size; and
      a second slot extending inwardly from said outer peripheral surface and adapted to couple with a syringe flange, said second slot defining at least one of a second shape different than said first shape and a second size different than said first size; and
   an actuator coupled to said adapter and including a plunger adapted to be advanced in a distal direction through said aperture in said proximal end wall to push fluid contained in the syringe.
Embodiment 19. The dispensing device with the features of embodiment 18, wherein said actuator is a non-manual actuator including an elongate body with a locking detent, and wherein said housing of said adapter further includes a plurality of locking arms extending proximally from said proximal end surface, said plurality of locking arms adapted to snap over said locking detent on said elongate body of said actuator to couple said adapter to said actuator.
Embodiment 20. The dispensing device with the features of embodiment 18, wherein said actuator is a manual actuator, and wherein said outer peripheral surface of said adapter further includes finger grip portions adapted to be grasped when said plunger of said actuator is manually actuated.
Embodiment 21. The dispensing device with the features of embodiment 18, wherein said first slot is positioned in said housing so as to be at least partially coextensive with said second slot.
Embodiment 22. The dispensing device with the features of embodiment 18, further comprising:
   a divider wall positioned between said first slot and said second slot, said divider wall including an internal aperture communicating with each of said first and second slots, said internal aperture adapted to receive the elongate body of the syringe.
Embodiment 23. The dispensing device with the features of embodiment 22, wherein said internal aperture includes a retention portion and a seat portion, said retention portion adapted to retain the elongate body of the syringe in said seat portion when the syringe is coupled to said adapter.
Embodiment 24. The dispensing device with the features of embodiment 18, wherein at least one of said first shape and said second shape is adapted to closely couple with syringe flanges including finger grip portions.
Embodiment 25. The dispensing device with the features of embodiment 18, wherein at least one of said first and second slots includes a retention portion and a seat portion, said retention portion adapted to retain the flange of the syringe in said seat portion when the syringe is coupled to said adapter.
Embodiment 26. A method of dispensing fluid with a dispensing device including an actuator having a plunger, the dispensing device further including an adapter including a housing with a first slot defining a first shape and a first size and a second slot defining at least one of a second shape different than the first shape and a second size different than the first size, the method comprising:
   inserting a flange of a first syringe into the first slot of the adapter to operatively couple the first syringe to the actuator;
   moving the plunger of the actuator to dispense fluid from the first syringe;
   removing the flange of the first syringe from the first slot to decouple the first syringe from the actuator;
   inserting a flange of a second syringe into the second slot of the adapter to operatively couple the second syringe to the actuator; and
   moving the plunger of the actuator to dispense fluid from the second syringe.
Embodiment 27. An adapter for coupling an actuator to a syringe including an elongate body and a flange, the adapter comprising:
   a housing including a proximal end wall, a distal end wall, and a side wall extending between said proximal and distal end walls and defining an outer peripheral surface, said proximal end wall adapted to face toward the actuator, said distal end wall adapted to face toward the syringe;
   a first slot extending inwardly from said outer peripheral surface and defining a first shape and a first size adapted to closely couple with a syringe flange including finger grip portions.
An adapter for a dispensing device is adapted to couple an actuator to a syringe including an elongate body and a flange. The adapter includes a housing including a proximal end wall, a distal end wall, and a side wall. The adapter includes first and second slots extending inwardly from the side wall, each slot defining at least one of differing shapes and differing sizes. The first and second slots may be at least partially coextensive or may be separated by a divider wall. The first and second slots may be shaped to couple with circular flanges, flanges with flats, or flanges including finger grip portions. Thus, the adapter may couple various syringes to the actuator.

## Claims

1. An adapter (14) for coupling an actuator (12) to a syringe (16) including an elongate body (18) and a flange (22), the adapter (14) comprising:
a housing (50) including a proximal end wall (52), a distal end wall (54), and a side wall (56) extending between said proximal and distal end walls (52, 54) and defining an outer peripheral surface (60), said distal end wall (54) adapted to face toward the syringe (16);
a first slot (30) extending inwardly from said outer peripheral surface (60) and adapted to couple with a syringe flange (22), said first slot (30) defining a first shape and a first size; and
a second slot (32) extending inwardly from said outer peripheral surface (60) and adapted to couple with a syringe flange (22), said second slot (32) defining a second size different than said first size,
**characterised in that** said second slot (32) defining also a second shape different than said first shape.

2. The adapter (14) of claim 1, wherein at least one of said first shape and said second shape is U-shaped so as to closely couple with one of the following: a) circular syringe flanges (22): b) and syringe flanges (22); c) with syringe flanges (22) with finger grip portions.

3. The adapter (14) of claim 1, wherein said first slot (30) is positioned in said housing (50) so as to be at least partially coextensive with said second slot (32).

4. The adapter (14) of claim 1, further comprising:
a divider wall (70) positioned between said first slot (30) and said second slot (32), said divider wall (70) including an internal aperture (74) communicating with each of said first and second slots (30, 32), said internal aperture (74) adapted to receive the elongate body (18) of the syringe (16).

5. The adapter (14) of claim 4, wherein said internal aperture (74) includes a retention portion (74a) and a seat portion (74b), said retention portion (74a) adapted to retain the elongate body (18) of the syringe (16) in said seat portion (74b) when the syringe (16) is coupled to the adapter (14).

6. The adapter (14) of claim 1, further comprising:
a third slot (34) extending inwardly from said outer peripheral surface (60) and adapted to couple with a syringe flange (22), said third slot (34) defining a third shape different than said first shape and said second shape, a third size different than said first size and said second size, or both said third shape and said third size.

7. The adapter (14) of claim 6, wherein said first, second, and third slots (30, 32, 34) are positioned in order of increasing size between said proximal end wall (52) and said distal end wall (54) of said housing (50).

8. The adapter (14) of claim 6, further comprising:
a divider wall (70) positioned between said first slot (30) and said second slot (32), said divider wall (70) including an internal aperture (74) communicating with each of said first and second slots (30, 32), said internal aperture (74) adapted to receive the elongate body (18) of the syringe (16),
wherein said second slot (32) is positioned in said housing (50) so as to be partially coextensive with said third slot (34).

9. The adapter (14) of claim 1, wherein said proximal end wall (52) of said housing (50) includes an aperture (68) adapted to receive a plunger (26) of an actuator (12), and said distal end wall (54) of said housing (50) includes an opening (69) in communication with said aperture (68) and adapted to receive the elongate body (18) of the syringe (16).

10. The adapter (14) of claim 9, wherein said opening (69) includes a retention portion (69a) and a seat portion (69b), said retention portion adapted to retain the elongate body (18) of the syringe (16) in said seat portion when the syringe (16) is coupled to the adapter (14).

11. The adapter (14) of claim 1, wherein the outer peripheral surface (60) of the housing (50) further includes one of the following: a) finger grip portions adapted to be grasped when the plunger (26) of the actuator (12) is manually actuated; or b) finger grip portions, the finger grip portions being removable from the housing (50) when the plunger (26) of the actuator (12) is to be non-manually actuated.

12. The adapter (14) of claim 1, wherein at least one of said first and second slots (30, 32) includes a retention portion (30a) and a seat portion (30b), said retention portion adapted to retain the flange (22) of the syringe (16) in said seat portion when the syringe (16) is coupled to the adapter (14).

13. A dispensing device (10) for dispensing fluid contained in a syringe (16) having an elongate body (18) and a flange (22), the dispensing device (10) comprising:
the adapter (14) according to any of the above claims;
a housing (50) including a proximal end wall (52), a distal end wall (54), and a side wall (56) extending between said proximal and distal end walls (52, 54) and defining an outer peripheral surface (60), said distal end wall (54) adapted to face toward the syringe (16);
a first slot (30) extending inwardly from said outer peripheral surface (60) and adapted to couple with a syringe flange (22), said first slot (30) defining a first shape and a first size; and
a second slot (32) extending inwardly from said outer peripheral surface (60) and adapted to couple with a syringe flange (22), said second slot (32) defining at least one of a second shape different than said first shape and a second size different than said first size; and
an actuator (12) coupled to said adapter (14) and including a plunger (26) adapted to be advanced in a distal direction to push fluid contained in the syringe (16).

14. The dispensing device (10) of claim 13, wherein said actuator (12) is a non-manual actuator including an elongate body (18) with a locking detent (66), and wherein said housing (50) of said adapter (14) further includes a plurality of locking arms (64) extending proximally from said proximal end surface, said plurality of locking arms (64) adapted to snap over said locking detent (66) on said elongate body (18) of said actuator (12) to couple said adapter (14) to said actuator (12),

15. A non-medical method of dispensing fluid with a dispensing device (210) including an actuator (212) having a plunger (258), the dispensing device (210) further including an adapter (214) including a housing (250) with a first slot (276) defining a first shape and a first size and a second slot (278) defining at least a second shape different than the first shape and a second size different than the first size, the method comprising:
inserting a flange (226) of a first syringe (216) into the first slot (276) of the adapter (214) to operatively couple the first syringe (216) to the actuator (212);
moving the plunger (258) of the actuator (212) to dispense fluid from the first syringe (216);
removing the flange (226) of the first syringe (216) from the first slot (276) to decouple the first syringe (216) from the actuator (212);
inserting a flange (236) of a second syringe (218) into the second slot (278) of the adapter (214) to operatively couple the second syringe (218) to the actuator (212); and
moving the plunger (258) of the actuator (212) to dispense fluid from the second syringe (218).

## Patentansprüche

1. Ein Adapter (14) zum Koppeln eines Aktuators (12) mit einer Spritze (16), welche einen länglichen Körper (18) und einen Flansch (22) beinhaltet, wobei der Adapter (14) umfaßt:
ein Gehäuse (50), welches eine proximale Endwand (52), eine distale Endwand (54), und eine Seitenwand (56), welche sich zwischen der proximalen und der distalen Endwand (52,54) erstreckt und eine äußere periphere Oberfläche (60) definiert, beinhaltet, wobei die distale Endwand (54) ausgebildet ist, um der Spritze (16) zugewandt zu sein;
einen ersten Schlitz (30), welcher sich von der äußeren peripheren Oberfläche (60) nach innen erstreckt und ausgebildet ist, um mit einem Spritzen-Flansch (22) zu koppeln, wobei der erste Schlitz (30) eine erste Form und eine erste Größe definiert; und
einen zweiten Schlitz (32), welcher sich von der äußeren peripheren Oberfläche (60) nach innen erstreckt und ausgebildet ist, um mit einem Spritzen-Flansch (22) zu koppeln, wobei der zweite Schlitz (32) eine zweite Größe definiert, welche unterschiedlich zu der ersten Größe ist,
**dadurch gekennzeichnet, dass** der zweite Schlitz (32) ebenfalls eine zweite Form definiert, welche unterschiedlich zu der ersten Form ist.

2. Der Adapter (14) gemäß Anspruch 1, wobei die erste Form und/oder die zweite Form U-förmig ausgebildet ist, um nah mit einem der folgenden zu koppeln: a) runden Spritzen-Flanschen (22); b) und Spritzen-Flanschen (22); c) mit Spritzen-Flanschen (22) mit Fingergriffabschnitten.

3. Der Adapter (14) gemäß Anspruch 1, wobei der erste Schlitz (30) in dem Gehäuse (50) positioniert ist, um zumindest teilweise mit dem zweiten Schlitz (32) zusammenzufallen.

4. Der Adapter (14) gemäß Anspruch 1, ferner umfassend:
eine Trennwand (70), welche zwischen dem ersten Schlitz (30) und dem zweiten Schlitz (32) positioniert ist, wobei die Trennwand (70) eine interne Öffnung (74) beinhaltet, welche mit dem ersten und dem zweiten Schlitz (30, 32) kommuniziert, wobei die interne Öffnung (74) ausgebildet ist, um den länglichen Körper (18) der Spritze (16) aufzunehmen.

5. Der Adapter (14) gemäß Anspruch 4, wobei die interne Öffnung (74) einen Zurückhaltungsabschnitt (74a) und einen Sitz-Abschnitt (74b) beinhaltet, wobei der Zurückhaltungsabschnitt (74a) ausgebildet ist, um den länglichen Körper (18) der Spritze (16) in dem Sitz-Abschnitt (74b) zu halten, wenn die Spritze (16) mit dem Adapter (14) gekoppelt ist.

6. Der Adapter (14) gemäß Anspruch 1, ferner umfassend:
einen dritten Schlitz (34), welcher sich von der äußeren peripheren Fläche (60) nach innen erstreckt und ausgebildet ist, um mit einem Spritzen-Flansch (22) zu koppeln, wobei der dritte Schlitz (34) eine dritte Form, welche unterschiedlich zu der ersten Form und zu der zweiten Form ist, eine dritte Größe, welche unterschiedlich zu der ersten Größe und zu der zweiten Größe ist, oder die dritte Form und die dritte Größe definiert.

7. Der Adapter (14) gemäß Anspruch 6, wobei der erste, zweite und dritte Schlitz (30, 32, 34) in der Reihenfolge ansteigender Größe zwischen der proximalen Endwand (52) und der distalen Endwand (54) des Gehäuses (50) positioniert sind.

8. Der Adapter (14) gemäß Anspruch 6, ferner umfassend:
eine Trennwand (70), welche zwischen dem ersten Schlitz (30) und dem zweiten Schlitz (32) positioniert ist, wobei die Trennwand (70) eine interne Öffnung (74) beinhaltet, welche mit dem ersten und zweiten Schlitz (30, 32) kommuniziert, wobei die interne Öffnung (74) ausgebildet ist, um den länglichen Körper (18) der Spritze (16) aufzunehmen,
wobei der zweite Schlitz (32) in dem Gehäuse (50) positioniert ist, um teilweise mit dem dritten Schlitz (34) zusammenzufallen.

9. Der Adapter (14) gemäß Anspruch 1, wobei die proximale Endwand (52) des Gehäuses (50) eine Öffnung (68) beinhaltet, welche ausgebildet ist, um einen Kolben (26) eines Aktuators (12) aufzunehmen, und die distale Endwand (54) des Gehäuses (50) eine Öffnung (69) in Kommunikation mit der Öffnung (68) beinhaltet und welche ausgebildet ist, um den länglichen Körper (18) der Spritze (16) aufzunehmen.

10. Der Adapter (14) gemäß Anspruch 9, wobei die Öffnung (69) einen Zurückhaltungsabschnitt (69a) und einen Sitz-Abschnitt (69b) beinhaltet, wobei der Zurückhaltungsabschnitt ausgebildet ist, um den länglichen Körper (18) der Spritze (16) in dem Sitz-Abschnitt zu halten, wenn die Spritze (16) mit dem Adapter (14) gekoppelt ist.

11. Der Adapter (14) gemäß Anspruch 1, wobei die äußere periphere Oberfläche (60) des Gehäuses (50) ferner eines der folgenden umfasst: a) Fingergriffabschnitte, ausgebildet um gegriffen werden zu können, wenn der Kolben (26) des Aktuators (12) manuell betätigt wird; oder b) Fingergriffabschnitte, wobei die Fingergriffabschnitte entfernbar von dem Gehäuse (50) sind, wenn der Kolben (26) des Aktuators (12) nicht-manuell betätigt werden soll.

12. Der Adapter (14) gemäß Anspruch 1, wobei der erste und/oder der zweite Schlitz (30, 32) einen Zurückhaltungsabschnitt (30a) und einen Sitz-Abschnitt (30b) beinhaltet, wobei der Zurückhaltungsabschnitt ausgebildet ist, um den Flansch (22) der Spritze (16) in dem Sitz-Abschnitt zu halten, wenn die Spritze (16) mit dem Adapter (14) gekoppelt ist.

13. Ein Abgabegerät (10) zum Abgeben von Fluid, welches in einer Spritze (16) enthalten ist, welche einen länglichen Körper (18) und einen Flansch (22) hat, wobei das Abgabegerät (10) umfasst:
den Adapter (14) gemäß einem der obigen Ansprüche;
ein Gehäuse (50), welches eine proximale Endwand (52), eine distale Endwand (54), und eine Seitenwand (56), welche sich zwischen proximalen und der distalen Endwand (52, 54) erstreckt und eine äußere periphere Oberfläche (60) definiert, beinhaltet, wobei die distale Endwand (54) ausgebildet ist, um der Spritze (16) zugewandt zu sein;
einen ersten Schlitz (30), welcher sich von der äußeren peripheren Oberfläche (60) nach innen erstreckt und ausgebildet ist, um mit einem Spritzen-Flansch (22) zu koppeln, wobei der erste Schlitz (30) eine erste Form und eine erste Größe definiert;
einen zweiten Schlitz (32), welcher sich von der äußeren peripheren Oberfläche (60) nach innen erstreckt und ausgebildet ist, um mit einem Spritzen-Flansch (22) zu koppeln, wobei der zweite Schlitz (32) mindestens eine zweite Form, welche unterschiedlich zu der ersten Form ist und eine zweite Größe, welche unterschiedlich zu der ersten Größe ist, definiert;
einen Aktuator (12), welcher mit dem Adapter (14) gekoppelt ist und einen Kolben (26) beinhaltet, welcher ausgebildet ist, um sich in distaler Richtung fortzubewegen, um Fluid, welches in der Spritze (16) enthalten ist, zu befördern.

14. Das Abgabegerät (10) gemäß Anspruch 13, wobei der Aktuator (12) ein nichtmanueller Aktuator ist, welcher einen länglichen Körper (18) mit einer Verriegelungsrastung (66) beinhaltet, und wobei das Gehäuse (50) des Adapters (14) ferner eine Vielzahl von Verriegelungsarmen (64) beinhaltet, welche sich proximal von der proximalen Endoberfläche erstrecken, wobei die Vielzahl der Verriegelungsarme (64) ausgebildet ist, um über die Verriegelungsrastung (66) auf dem länglichen Körper (18) des Aktuators (12) zu schnappen, um den Adapter (14) mit den Aktuator (12) zu koppeln.

15. Ein nicht-medizinisches Verfahren zum Abgeben von Fluid mit einem Abgabegerät (210), welches einen Aktuator (212) beinhaltet, welcher einen Kolben (258) hat, wobei das Abgabegerät (210) ferner einen Adapter (214) beinhaltet, welcher ein Gehäuse (250) mit einem ersten Schlitz (276), welcher eine erste Form und eine erste Größe definiert und einen zweiten Schlitz (278), welcher mindestens eine zweite Form, welche unterschiedlich zu der ersten Form ist, und eine zweite Größe, welche unterschiedlich zu der ersten Größe ist, definiert, beinhaltet, das Verfahren umfassend:
Einsetzen eines Flansches (226) einer ersten Spritze (216) in den ersten Schlitz (276) des Adapters (214), um die erste Spritze (216) mit dem Aktuator (212) operativ zu koppeln;
Bewegen des Kolbens (258) des Aktuators (212), um Fluid aus der ersten Spritze (216) abzugeben;
Entfernen des Flansches (226) der ersten Spritze (216) von dem ersten Schlitz (276), um die erste Spritze (216) von dem Aktuator (212) zu entkoppeln;
Einsetzen eines Flansches (236) einer zweiten Spritze (218) in den zweiten Schlitz (278) des Adapters (214), um die zweite Spritze (218) mit dem Aktuator (212) operativ zu koppeln;
Bewegen des Kolbens (258) des Aktuators (212), um Fluid aus der zweiten Spritze (218) abzugeben.

## Revendications

1. Adaptateur (14) pour accoupler un actionneur (12) avec une seringue (16) comprenant un corps al longé (18) et une bride (22), l'adaptateur (14) comprenant :
un logement (50) comprenant une paroi d'extrémité proximale (52), une paroi d'extrémité distale (54), et une paroi latérale (56) s'étendant entre lesdites parois d'extrémité proximale et distale (52, 54) et définissant une surface périphérique extérieure (60), ladite paroi d'extrémité distale (54) étant adaptée pour être orientée vers la seringue (16) ;
une première fente (30) s'étendant vers l'intérieur à partir de ladite surface périphérique extérieure (60) et étant adaptée pour s'accoupler avec une bride de seringue (22), ladite première fente (30) définissant une première forme et une première taille ; et
une deuxième fente (32) s'étendant vers l'intérieur à partir de ladite surface périphérique extérieure (60) et étant adaptée pour s'accoupler avec une bride de seringue (22), ladite deuxième fente (32) définissant une deuxième taille différente de ladite première taille,
**caractérisé en ce que** ladite deuxième fente (32) définit également une deuxième forme différente de ladite première forme.

2. Adaptateur (14) selon la revendication 1, dans lequel au moins une parmi ladite première forme et ladite deuxième forme présente une forme de U afin de s'accoupler de près avec un des éléments suivants : a) des brides de seringue circulaires (22) ; b) des brides de seringue (22) ; c) des brides de seringue (22) avec des parties de préhension pour doigts.

3. Adaptateur (14) selon la revendication 1, dans lequel ladite première fente (30) est positionnée dans ledit logement (50) afin d'être au moins partiellement coextensive avec ladite deuxième fente (32).

4. Adaptateur (14) selon la revendication 1, comprenant en outre :
une paroi de séparation (70) positionnée entre ladite première fente (30) et ladite deuxième fente (32), ladite paroi de séparation (70) comprenant une ouverture interne (74) communiquant avec chacune desdites première et deuxième fentes (30, 32), ladite ouverture interne (74) étant adaptée pour recevoir le corps allongé (18) de la seringue (16).

5. Adaptateur (14) selon la revendication 4, dans lequel ladite ouverture interne (74) comprend une partie de retenue (74a) et une partie de siège (74b), ladite partie de retenue (74a) étant adaptée pour retenir le corps allongé (18) de la seringue (16) dans ladite partie de siège (74b) lorsque la seringue (16) est accouplée avec l'adaptateur (14).

6. Adaptateur (14) selon la revendication 1, comprenant en outre :
une troisième fente (34) s'étendant vers l'intérieur à partir de ladite surface périphérique extérieure (60) et étant adaptée pour s'accoupler avec une bride de seringue (22), ladite troisième fente (34) définissant une troisième forme différente de ladite première forme et de ladite deuxième forme, une troisième taille différente de ladite première taille et de ladite deuxième taille, ou à la fois ladite troisième forme et ladite troisième taille.

7. Adaptateur (14) selon la revendication 6, dans lequel lesdites première, deuxième et troisième fentes (30, 32, 34) sont positionnées dans un ordre croissant en taille entre ladite paroi d'extrémité proximale (52) et ladite paroi d'extrémité distale (54) dudit logement (50).

8. Adaptateur (14) selon la revendication 6, comprenant en outre :
une paroi de séparation (70) positionnée entre ladite première fente (30) et ladite deuxième fente (32), ladite paroi de séparation (70) comprenant une ouverture interne (74) communiquant avec chacune desdites première et deuxième fentes (30, 32), ladite ouverture interne (74) étant adaptée pour recevoir le corps allongé (18) de la seringue (16),
dans lequel ladite deuxième fente (32) est positionnée dans ledit logement (50) afin d'être partiellement coextensive avec ladite troisième fente (34).

9. Adaptateur (14) selon la revendication 1, dans lequel ladite paroi d'extrémité proximale (52) dudit logement (50) comprend une ouverture (68) adaptée pour recevoir un piston plongeur (26) d'un actionneur (12), et ladite paroi d'extrémité distale (54) dudit logement (50) comprend une fenêtre (69) en communication avec ladite ouverture (68) et adaptée pour recevoir le corps allongé (18) de la seringue (16).

10. Adaptateur (14) selon la revendication 9, dans lequel ladite fenêtre (69) comprend une partie de retenue (69a) et une partie de siège (69b), ladite partie de retenue étant adaptée pour retenir le corps allongé (18) de la seringue (16) dans ladite partie de siège lorsque la seringue (16) est accouplée avec l'adaptateur (14).

11. Adaptateur (14) selon la revendication 1, dans lequel la surface périphérique extérieure (60) du logement (50) comprend en outre un des éléments suivants : a) des parties de préhension pour doigts adaptées pour être saisies lorsque le piston plongeur (26) de l'actionneur (12) est actionné manuellement ; ou b) des parties de préhension pour doigts, les parties de préhension pour doigts étant amovibles à partir du logement (50) lorsque le piston plongeur (26) de l'actionneur (12) doit être actionné non manuellement.

12. Adaptateur (14) selon la revendication 1, dans lequel au moins une desdites première et deuxième fentes (30, 32) comprend une partie de retenue (30a) et une partie de siège (30b), ladite partie de retenue étant adaptée pour retenir la bride (22) de la seringue (16) dans ladite partie de siège lorsque la seringue (16) est accouplée avec l'adaptateur (14).

13. Dispositif de distribution (10) pour distribuer un fluide contenu dans une seringue (16) possédant un corps allongé (18) et une bride (22), le dispositif de distribution (10) comprenant :
l'adaptateur (14) selon une quelconque des revendications précédentes ;
un logement (50) comprenant une paroi d'extrémité proximale (52), une paroi d'extrémité distale (54), et une paroi latérale (56) s'étendant entre lesdites parois d'extrémité proximale et distale (52, 54) et définissant une surface périphérique extérieure (60), ladite paroi d'extrémité distale (54) étant adaptée pour être orientée vers la seringue (16) ;
une première fente (30) s'étendant vers l'intérieur à partir de ladite surface périphérique extérieure (60) et étant adaptée pour s'accoupler avec une bride de seringue (22), ladite première fente (30) définissant une première forme et une première taille ; et
une deuxième fente (32) s'étendant vers l'intérieur à partir de ladite surface périphérique extérieure (60) et étant adaptée pour s'accoupler avec une bride de seringue (22), ladite deuxième fente (32) définissant au moins une d'une deuxième forme différente de ladite première forme et une deuxième taille différente de ladite première taille ; et
un actionneur (12) accouplé avec ledit adaptateur (14) et comprenant un piston plongeur (26) adapté pour être avancé dans une direction distale pour pousser un fluide contenu dans la seringue (16).

14. Dispositif de distribution (10) selon la revendication 13, dans lequel ledit actionneur (12) est un actionneur non manuel comprenant un corps allongé (18) avec un cliquet de verrouillage (66), et dans lequel ledit logement (50) dudit adaptateur (14) comprend en outre une pluralité de bras de verrouillage (64) s'étendant de façon proximale à partir de ladite surface d'extrémité proximale, ladite pluralité de bras de verrouillage (64) étant adaptés pour s'encliqueter sur ledit cliquet de verrouillage (66) sur ledit corps allongé (18) dudit actionneur (12) pour accoupler ledit adaptateur (14) avec ledit actionneur (12).

15. Procédé non médical de distribution de fluide avec un dispositif de distribution (210) comprenant un actionneur (212) possédant un piston plongeur (258), le dispositif de distribution (210) comprenant en outre un adaptateur (214) comprenant un logement (250) avec une première fente (276) définissant une première forme et une première taille et une deuxième fente (278) définissant au moins une deuxième forme différente de la première forme et une deuxième taille différente de la première taille, le procédé comprenant :
l'insertion d'une bride (226) d'une première seringue (216) dans la première fente (276) de l'adaptateur (214) pour accoupler fonctionnellement la première seringue (216) avec l'actionneur (212) ;
le déplacement du piston plongeur (258) de l'actionneur (212) pour distribuer un fluide à partir de la première seringue (216) ;
le retrait de la bride (226) de la première seringue (216) à partir de la première fente (276) pour désaccoupler la première seringue (216) de l'actionneur (212) ;
l'insertion d'une bride (236) d'une seconde seringue (218) dans la deuxième fente (278) de l'adaptateur (214) pour accoupler fonctionnellement la seconde seringue (218) avec l'actionneur (212) ; et
le déplacement du piston plongeur (258) de l'actionneur (212) pour distribuer un fluide à partir de la seconde seringue (218).
